# EUROPEAN PATENT SPECIFICATION

(11) **EP 0 799 246 B1**
(45) Date of publication and mention of the grant of the patent: **15.11.2000**
(21) Application number: 95941531.6
(22) Date of filing: 13.12.1995
(51) Int. Cl.: C08B 37/00, G03G 9/09, G03G 9/097

(54) **IMPROVED MUTABLE COMPOSITION**
VERBESSERTE, ÄNDERBARE ZUSAMMENSETZUNG
COMPOSITION MUTABLE AMELIOREE

(30) Priority: 20.12.1994 US 359670
(43) Date of publication of application: 08.10.1997
(60) Divisional application: 00104842.0
(73) Proprietor: Kimberly-Clark Worldwide, Inc., Neenah, WI 54957-0349 (US)
(72) Inventor: NOHR, Ronald, Sinclair, Alpharetta, GA 30202 (US); MacDONALD, John, Gavin, Decatur, GA 30033 (US)
(74) Representative: DIEHL GLAESER HILTL & PARTNER
(86) International application number: US9516137
(87) International publication number: WO9619502

(56) References cited:
- WO-A-95/04955
- DE-A- 4 126 461
- PATENT ABSTRACTS OF JAPAN vol. 13, no. 15 (C-559), 13 January 1989 & JP,A,63 223084 (AGENCY OF IND SCIENCE), 16 September 1988,
- PATENT ABSTRACTS OF JAPAN vol. 8, no. 30 (C-209), 8 February 1984 & JP,A,58 194835 (HIDEFUMI HIRAI), 12 November 1983,
- PATENT ABSTRACTS OF JAPAN vol. 13, no. 241 (C-604), 6 June 1989 & JP,A,01 051402 (TOSOH), 27 February 1989,
- PATENT ABSTRACTS OF JAPAN vol. 13, no. 516 (C-656), 17 November 1989 & JP,A,01 210477 (CANON), 24 August 1989,
- DATABASE WPI Section Ch, Week 9021 Derwent Publications Ltd., London, GB; Class G05, AN 90-159060 XP002001469 & JP,A,02 099 384 (RICOH) , 11 April 1990
- DATABASE WPI Section Ch, Week 9445 Derwent Publications Ltd., London, GB; Class G02, AN 94-363806 XP002001470 & JP,A,06 287 497 (PILOT INK) , 11 October 1994
- DATABASE WPI Section Ch, Week 8724 Derwent Publications Ltd., London, GB; Class G06, AN 87-167588 XP002001471 & JP,A,62 100 557 (MITSUBISHI) , 11 May 1987

## Description

### Background of the Invention

The present invention relates to a colored composition, a method of making a molecular includant, and a functionalized molecular includant. In some embodiments, the colored composition may be employed in an electrographic toner, e.g., a toner employed in a photocopier which is based on transfer xerography.

The Application Serial US No. 08/258,858 (=WO 9504955 published 16.02.95) describes a colored composition which includes a colorant and an ultraviolet radiation transorber. The colorant, in the presence of the ultraviolet radiation transorber, is adapted, upon exposure of the transorber to ultraviolet radiation, to be mutable. The ultraviolet radiation transorber is adapted to absorb ultraviolet radiation and interact with the colorant to effect the irreversible mutation of the colorant. The ultraviolet radiation in general will have a wavelength of from about 4 to about 400 nanometers. Especially useful ultraviolet radiation is incoherent, pulsed ultraviolet radiation produced by a dielectric barrier discharge excimer lamp.

The colored composition also may contain a molecular includant having a chemical structure which defines at least one cavity. Each of the colorant and ultraviolet radiation transorber is associated with the molecular includant. For example, the colorant may be at least partially included within a cavity of the molecular includant and the ultraviolet radiation transorber may be associated with the molecular includant outside of the cavity. As another example, the ultraviolet radiation transorber may be covalently coupled to the molecular includant.

From DE 41 26 461 A1 a colorant coupled inorganic molecular sieve is known. These molecular sieves can be applied as pigments in colors as well as optical data saving substances for memories.

The JP-A-63223084 suggests a photochromic composition wherein a specific spirobenzophran compound is included in a modified gamma-cyclodextrin.

From JP-A-6287497 a color-dying making pen ink is known. The pen ink consists of a solution obtained by making a pH indicator produce color with a basic substance and containing at least one cyclodextrin or cyclodextrin derivative.

JP-A-58194835 describes the synthesis of 4-Allyl-2,5-Cyclohexadienone derivatives which can be useful as a raw material for synthesizing a physiologically active substance.

JP-A-2099384 teaches the improvement of coloring sensitivity and shelf life of priming brightness by a method wherein a multimolecular organic enclosure compound is contained in a thermal coloring layer.

From JP-A-1210477 a recording fluid and recording method are known. The recording fluid is obtained essentially by mixing beta-cylodextrin with a water-soluble ultraviolet absorber.

None of the above documents provide a covalent bonding between the ultraviolet radiation transorber and a molecular includant.

In those embodiments in which the ultraviolet radiation transorber is covalently coupled to the molecular includant, the efficiency of energy transfer from the ultraviolet radiation transorber to the colorant is, at least in part, a function of the number of ultraviolet radiation transorber molecules which are attached to the molecular includant. It now is known that the synthetic methods described in the above Application Serial No. 08/258,858 resulted in covalently coupling an average of two transorber molecules to each molecule of the molecular includant. Because the time required to mutate the colorant should be, at least in part, a function of the number of ultraviolet radiation transorber molecules coupled to each molecule of molecular includant, there is a need for an improved colored composition in which an average of more than two ultraviolet radiation transorber molecules are covalently coupled to each mblecule of the molecular includant.

### Summary of the Invention

The present invention solves the above objects by the subject-matter of independent claims 1,10,12,15,16, and 19. Preferred embodiments are described in the dependent claims.

The present invention further addresses some of the difficulties and problems discussed above by providing a mutable composition which includes a colorant, an ultraviolet radiation transorber, and a molecular includant wherein more than two molecules of the ultraviolet radiation transorber are covalently coupled to each molecule of the molecular includant. For convenience, the combination of ultraviolet radiation transorber and molecular includant is referred to hereinafter as a functionalized molecular includant. That is the functionalized molecular includant is a molecular includant to which more than two molecules of an ultraviolet radiation transorber are covalently coupled to each molecule of the molecular includant. Thus, the mutable composition of the present invention includes a colorant and a functionalized molecular includant as defined above.

The colorant, in the presence of the functionalized molecular includant, is adapted, upon exposure of the functionalized molecular includant to ultraviolet radiation, to be mutable. It is desirable that the mutation of the colorant be irreversible. The ultraviolet radiation transorber molecules covalently coupled to the molecular includant molecules are adapted to absorb ultraviolet radiation and interact with, and to effect the irreversible mutation of, the colorant. It is desirable that the ultraviolet radiation transorber absorb ultraviolet radiation at a wavelength of from about 4 to about 400 nanometers. It is even more desirable that the ultraviolet radiation transorber absorb ultraviolet radiation at a wavelength of 100 to 375 nanometers. Especially useful ultraviolet radiation is incoherent, pulsed ultraviolet radiation produced by a dielectric barrier discharge excimer lamp.

The molecular includant has a chemical structure which defines at least one cavity. Molecular includants include, but are not limited to, clathrates, zeolites, and cyclodextrins.

The colorant is associated with the functionalized molecular includant. The term "associated" in its broadest sense means that the colorant is at least in close proximity to the functionalized molecular includant. For example, the colorant may be maintained in close proximity to the functionalized molecular includant by hydrogen bonding, van der Waals forces, or the like. Alternatively, the colorant may be covalently bonded to the functionalized molecular includant, although this normally is neither desired nor necessary. As a further example, the colorant may be at least partially included within the cavity of the functionalized molecular includant.

The ultraviolet radiation transorber may be any material which is adapted to absorb ultraviolet radiation and interact with the colorant to effect the mutation of the colorant. For example, the ultraviolet radiation transorber may be an organic compound. As another example, the ultraviolet radiation transorber may be a single material or a mixture of two or more materials. If two or more materials are employed, it is not necessary that all of them absorb ultraviolet radiation of the same wavelength.

The ultraviolet radiation transorber may include a known photoreactor or photoinitiator in combination with a material adapted to shift the absorption maximum of the photoreactor, i.e., a wavelength-selective sensitizer. The wavelength-selective sensitizer may be covalently coupled to the photoreactor. By way of example, the wavelength-selective sensitizer may be selected from the group consisting of phthaloylglycine and 4-(4-oxyphenyl)-2-butanone. As another example, the photoreactor may be selected from the group consisting of 1-[4-(2-hydroxyethoxy)phenyl]-2-hydroxy-2-methylpropan-1-one and cyclohexyl-phenyl ketone ester. As a further example, the ultraviolet radiation transorber may be 2-[p-(2-methyllactoyl)phenoxy]ethyl 1,3-dioxo-2-isoindolineacetate. As still another example, the ultraviolet radiation transorber may be 2-hydroxy-2-methyl-4'-[2-[p-(3-oxobutyl)phenoxy]ethoxy]propiophenone.

The present invention also provides a method of making a functionalized molecular includant which involves the steps of providing a derivatized ultraviolet radiation transorber having a nucleophilic group, providing a derivatized molecular includant having more than two leaving groups per molecule, and reacting the derivatized ultraviolet radiation transorber with the derivatized molecular includant under conditions sufficient to result in the covalent coupling of an average of more than two ultraviolet radiation transorber molecules to each molecular includant molecule. By way of example, the derivatized ultraviolet radiation transorber may be 2-[p-(2-methyl-2-mercaptomethylpropionyl)phenoxy]ethyl 1,3-dioxo-2-isoindolineacetate. As another example, the derivatized ultraviolet radiation transorber may 2-mercaptomethyl-2-methyl-4'-[2-[p-(3-oxobutyl)phenoxy]ethoxy]propiophenone.

### Brief Description of the Drawings

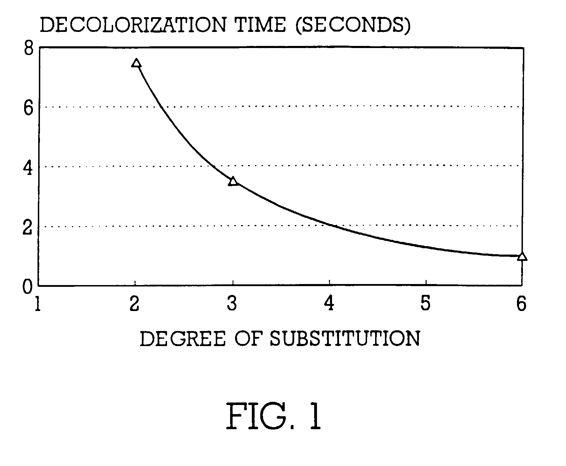

FIG. 1 is a plot of the average number of ultraviolet radiation transorber molecules which are covalently coupled to each molecule of a molecular includant in several colored compositions, which number also is referred to by the term, "degree of substitution," versus the decolorization time upon exposure to 222-nanometer excimer lamp ultraviolet radiation.

### Detailed Description of the Invention

The present invention relates in general to a colorant system that is mutable by exposure to radiation. More particularly, the present invention relates to a composition which includes a colorant and a functionalized molecular includant.

The term "functionalized molecular includant" is used herein to mean a molecular includant to which more than two molecules of an ultraviolet radiation transorber are covalently coupled to each molecule of the molecular includant. The term "degree of substitution" is used herein to refer to the number of ultraviolet radiation transorber molecules or leaving groups which are covalently coupled to each molecule of the molecular includant. For example, the degree of substitution of the functionalized molecular includant may be in a range of from 3 to about 21. As another example, the degree of substitution may be in a range of from 3 to about 10. As a further example, the degree of substitution may be in a range of from about 4 to about 9.

The term "molecular includant," as used herein, is intended to mean any substance having a chemical structure which defines at least one cavity. That is, the molecular includant is a cavity-containing structure. As used herein, the term "cavity" is meant to include any opening or space of a size sufficient to accept at least a portion of the colorant. Thus, the cavity can be a tunnel through the molecular includant or a cave-like space in the molecular includant. The cavity can be isolated or independent, or connected to one or more other cavities.

The molecular includant can be inorganic or organic in nature. In certain embodiments, the chemical structure of the molecular includant is adapted to form a molecular inclusion complex. Examples of molecular includants are, by way of illustration only, clathrates or intercalates, zeolites, and cyclodextrins. Examples of cyclodextrins include, but are not limited to, alpha-cyclodextrin (α-cyclodextrin), beta-cyclodextrin (β-cyclodextrin), gamma-cyclodextrin, hydroxypropyl β-cyclodextrin, hydroxyethyl β-cyclodextrin, sulfated β-cyclodextrin, and sulfated gamma-cyclodextrin (American Maize Products Company, of Hammond Indiana). In some embodiments, the molecular includant is a cyclodextrin. For example, the molecular includant may be an α-cyclodextrin. As another example, the molecular includant may be a β-cyclodextrin.

As used herein, the term "derivatized molecular includant" is used herein to mean a molecular includant having more than two leaving groups covalently coupled to each molecular of the molecular includant. The term "leaving group" is used herein to mean any leaving group capable of participating in a bimolecular nucleophilic substitution reaction.

The colorant, in the presence of the functionalized molecular includant, is adapted, upon exposure of the functionalized molecular includant to ultraviolet radiation, to be mutable. The ultraviolet radiation transorber is capable of absorbing radiation and interacting with the colorant to effect a mutation of the colorant.

The term "composition" and such variations as "colored composition" are used herein to mean a colorant and a functionalized molecular includant. When reference is being made to a colored composition which is adapted for a specific application, such as a toner to be used in an electrophotographic process, the term "composition-based" is used as a modifier to indicate that the material, e.g., a toner, includes a colorant and a functionalized molecular includant.

As used herein, the term "colorant" is meant to include, without limitation, any material which, in the presence of a functionalized molecular includant, is adapted upon exposure to ultraviolet radiation to be mutable. The colorant typically will be an organic material, such as an organic dye or pigment, including toners and lakes. Desirably, the colorant will be substantially transparent to, that is, will not significantly interact with, the ultraviolet radiation to which it is exposed. The term is meant to include a single material or a mixture of two or more materials which may be of the same or different types.

Organic dye classes include, by way of illustration only, triaryl methyl dyes, such as Malachite Green Carbinol base {4-(dimethylamino)-α-[4-(dimethylamino)phenyl]-α-phenylbenzene-methanol}, Malachite Green Carbinol hydrochloride {N-4-[[4-(dimethylamino)phenyl]-phenylmethylene]-2,5-cyclohexyldien-1-ylidene]-N-methyl-methanaminium chloride or bis[p-(dimethylamino)phenyl]phenylmethylium chloride}, Malachite Green oxalate {N-4-[[4-(dimethylamino)phenyl]phenylmethylene]-2,5-cyclohexyldien-1-ylidene]-N-methylmethanaminium chloride or bis[p-(dimethylamino)phenyl]phenylmethylium oxalate}, Victoria Pure Blue BO {N-[4-[[4-diethylamino)phenyl]-[4-(ethylamino)-1-naphthalenyl]methylene]-2,5-cyclohexadien-1-yliden]-N-ethyl-ethanaminium chloride}, and Basic Fusion {4-[(4-aminophenyl)-(4-imino-2,5-cyclohexadien-1-ylidene)methyl]benzenamine monohydrochloride}; monoazo dyes, such as Cyanine Black, Chrysoidine [Basic Orange 2; 4-(phenylazo)-1,3-benzenediamine monohydrochloride], and β-Naphthol Orange; thiazine dyes, such as Methylene Green, zinc chloride double salt [3,7-bis(dimethylamino)-6-nitrophenothiazin-5-ium chloride, zinc chloride double salt]; oxazine dyes, such as Lumichrome (7,8-dimethylalloxazine); naphthalimide dyes, such as Lucifer Yellow CH {6-amino-2-[(hydrazinocarbonyl)amino]-2,3-dihydro-1,3-dioxo-1H-benz[de]isoquinoline-5,8-disulfonic acid dilithium salt}; azine dyes, such as Janus Green B {3-(diethylamino)-7-[[4-(dimethylamino)phenyl]azo]-5-phenylphenazinium chloride}; cyanine dyes, such as Indocyanine Green {Cardio-Green or Fox Green; 2-[7-[1,3-dihydro-1,1-dimethyl-3-(4-sulfobutyl)-2H-benz[e]indol-2-ylidene]-1,3,5-heptatrienyl]-1,1-dimethyl-3-(4-sulfobutyl)-1H-benz[e]indolium hydroxide inner salt sodium salt}; indigo dyes, such as Indigo {Indigo Blue or Vat Blue 1; 2-(1,3-dihydro-3-oxo-2H-indol-2-ylidene)-1,2-dihydro-3H-indol-3-one}; coumarin dyes, such as 7-hydroxy-4-methylcoumarin (4-methylumbelliferone); benzimidazole dyes, such as Hoechst 33258 [bisbenzimide or 2-(4-hydroxyphenyl)-5-(4-methyl-1-piperazinyl)-2,5-bi-1H-benzimidazole trihydrochloride pentahydrate]; paraquinoidal dyes, such as Hematoxylin {Natural Black 1; 7,11b-dihydrobenz-[b]indeno[1,2-d]pyran-3,4,6a,9,10(6H)-pentol}; fluorescein dyes, such as Fluoresceinamine (5-aminofluorescein); diazonium salt dyes, such as Diazo Red RC (Azoic Diazo No. 10 or Fast Red RC salt; 2-methoxy-5-chlorobenzenediazonium chloride, zinc chloride double salt) ; azoic diazo dyes, such as Fast Blue BB salt (Azoic Diazo No. 20; 4-benzoylamino-2,5-diethoxybenzene diazonium chloride, zinc chloride double salt); phenylenediamine dyes, such as Disperse Yellow 9 [N-(2,4-dinitrophenyl)-1,4-phenylenediamine or Solvent Orange 53]; diazo dyes, such as Disperse Orange 13 [Solvent Orange 52; 1-phenylazo-4-(4-hydroxyphenylazo)naphthalene]; anthraquinone dyes, such as Disperse Blue 3 [Celliton Fast Blue FFR; 1-methylamino-4-(2-hydroxyethylamino)-9,10-anthraquinone], Disperse Blue 14 [Celliton Fast Blue B; 1,4-bis(methylamino)-9,10-anthraquinone], and Alizarin Blue Black B (Mordant Black 13); trisazo dyes, such as Direct Blue 71 {Benzo Light Blue FFL or Sirius Light Blue BRR; 3-[(4-[(4-[(6-amino-1-hydroxy-3-sulfo-2-naphthalenyl)azo]-6-sulfo-1-naphthalenyl)azo]-1-naphthalenyl)azo]-1,5-naphthalenedisulfonic acid tetrasodium salt}; xanthene dyes, such as 2,7-dichlorofluorescein; proflavine dyes, such as 3,6-diaminoacridine hemisulfate (Proflavine); sulfonaphthalein dyes, such as Cresol Red (o-cresolsulfonaphthalein); phthalocyanine dyes, such as Copper Phthalocyanine {Pigment Blue 15; (SP-4-1)-[29H,31H-phthalocyanato(2-)-N²⁹,N³⁰,N³¹,N³²]copper}; carotenoid dyes, such as trans-β-carotene (Food Orange 5); carminic acid dyes, such as Carmine, the aluminum or calcium-aluminum lake of carminic acid (7-α-D-glucopyranosyl-9,10-dihydro-3,5,6,8-tetrahydroxy-1-methyl-9,10-dioxo-2-anthracenecarboxylic acid); azure dyes, such as Azure A [3-amino-7-(dimethylamino)phenothiazin-5-ium chloride or 7-(dimethylamino)-3-imino-3H-phenothiazine hydrochloride]; and acridine dyes, such as Acridine Orange [Basic Orange 14; 3,8-bis(dimethylamino)acridine hydrochloride, zinc chloride double salt] and Acriflavine (Acriflavine neutral; 3,6-diamino-10-methylacridinium chloride mixture with 3,6-acridinediamine).

The term "mutable" with reference to the colorant is used to mean that the absorption maximum of the colorant in the visible region of the electromagnetic spectrum is capable of being mutated or changed by exposure to ultraviolet radiation when in the presence of the functionalized molecular includant. In general, it is only necessary that such absorption maximum be mutated to an absorption maximum which is different from that of the colorant prior to exposure to the ultraviolet radiation, and that the mutation be irreversible. Thus, the new absorption maximum can be within or outside of the visible region of the electromagnetic spectrum. In other words, the colorant may mutate to a different color or be rendered colorless. The latter, of course, is desirable when the colorant is used in a colored composition adapted to be utilized as a toner in an electrophotographic process which reuses the electrophotographic copy by first rendering the colored composition colorless and then placing a new image thereon.

As used herein, the term "irreversible" means that the colorant will not revert to its original color when it no longer is exposed to ultraviolet radiation. Desirably, the mutated colorant will be stable, i.e., not appreciably adversely affected by radiation normally encountered in the environment, such as natural or artificial light and heat. Thus, desirably, a colorant rendered colorless will remain colorless indefinitely.

The term "ultraviolet radiation transorber" is used herein to mean any material which is adapted to absorb ultraviolet radiation and interact with the colorant to effect the mutation of the colorant. For example, the ultraviolet radiation transorber may be an organic compound. Thus, the term includes the known photoreactors or photoinitiators. The term "material" is intended to include the term "compound" and to include a single material or a mixture of two or more materials. If two or more materials are employed, it is not necessary that all of them absorb ultraviolet radiation of the same wavelength.

The ultraviolet radiation transorber also may be a compound that is capable of absorbing narrow bandwidth ultraviolet wavelength radiation. For example, the ultraviolet radiation transorber may include a known photoreactor in combination with a material adapted to shift the absorption maximum of the photoreactor, i.e., a wavelength-selective sensitizer. The wavelength-selective sensitizer may be covalently coupled to the photoreactor. The known photoreactors oftentimes do not efficiently absorb high energy radiation. When combined with the wavelength-selective sensitizer, however, the resulting compound is an ultraviolet radiation transorber that efficiently absorbs a very narrow spectrum of radiation. By way of example, the wavelength-selective sensitizer may be selected from the group consisting of phthaloylglycines and 4-(4-oxyphenyl)-2-butanones. As another example, the photoreactor may be selected from the group consisting of 1-[4-(2-hydroxyethoxy)phenyl]-2-hydroxy-2-methylpropan-1-ones and cyclohexyl-phenyl ketone esters. As a further example, the ultraviolet radiation transorber may be 2-[p-(2-methyllactoyl)phenoxy]ethyl 1,3-dioxo-2-isoindolineacetate. As still another example, the ultraviolet radiation transorber may be 2-hydroxy-2-methyl-4'-[2-[p-(3-oxobutyl)phenoxy]ethoxy]propiophenone.

While the mechanism of the interaction of the ultraviolet radiation transorber with the colorant is not totally understood, it is believed that it may interact with the colorant in a variety of ways. For example, the ultraviolet radiation transorber, upon absorbing ultraviolet radiation, may be converted to one or more free radicals which interact with the colorant. Such free radical-generating compounds typically are hindered ketones, some examples of which are benzildimethyl ketal (available commercially as Irgacure® 651, Ciba-Geigy Corporation, Hawthorne, New York), 1-hydroxycyclohexyl phenyl ketone (Irgacure® 500), 2-methyl-1-[4-(methylthio)phenyl]-2-morpholino-propan-1-one] (Irgacure® 907), 2-benzyl-2-dimethylamino-l-(4-morpholinophenyl)butan-1-one (Irgacure® 369), and 1-hydroxycyclohexyl phenyl ketone (Irgacure® 184).

Alternatively, the ultraviolet radiation may initiate an electron transfer or reduction-oxidation reaction between the ultraviolet radiation transorber and the colorant. In this case, the ultraviolet radiation transorber may be Michler's ketone (p-dimethylaminophenyl ketone) or benzyl trimethyl stannate. Or, a cationic mechanism may be involved, in which case the ultraviolet radiation transorber may be, for example, bis[4-(diphenylsulphonio)phenyl)] sulfide bis(hexafluorophosphate) (Degacure® KI85, Ciba-Geigy Corporation, Hawthorne, New York); Cyracure® UVI-6990 (Ciba-Geigy Corporation), a mixture of bis[4-(diphenylsulphonio)phenyl] sulfide bis(hexafluorophosphate) with related monosulphonium hexafluorophosphate salts; and η⁵-2,4-(cyclopentadienyl)[1,2,3,4,5,6-η-(methylethyl)benzene]-iron(II) hexafluorophosphate (Irgacure® 261).

The term "ultraviolet radiation" is used herein to mean electromagnetic radiation having wavelengths in the range of from about 4 to about 400 nanometers. The especially desirable ultraviolet radiation range for the present invention is from about 100 to about 375 nanometers. Thus, the term includes the regions commonly referred to as ultraviolet and vacuum ultraviolet. The wavelength ranges typically assigned to these two regions are from about 180 to about 400 nanometers and from about 100 to about 180 nanometers, respectively.

In some embodiments, the molar ratio of ultraviolet radiation transorber (covalently coupled to the molecular includant) to colorant generally will be equal to or greater than about 0.5. As a general rule, the more efficient the ultraviolet radiation transorber is in absorbing the ultraviolet radiation and interacting with, i.e., transferring absorbed energy to, the colorant to effect irreversible mutation of the colorant, the lower such ratio can be. Current theories of molecular photo chemistry suggest that the lower limit to such ratio is 0.5, based on the generation of two free radicals per photon. As a practical matter, however, ratios higher than 1 are likely to be required, perhaps as high as about 10. However, the present invention is not bound by any specific molar ratio range. The important feature is that the ultraviolet radiation transorber is present in an amount sufficient to effect mutation of the colorant.

As a practical matter, the colorant and functionalized molecular includant are likely to be solids. However, any or all of such materials can be a liquid. For example, the colored composition may be a liquid because one or more of its components is a liquid, or, when the molecular includant is organic in nature, a solvent is employed. Suitable solvents include, but are not limited to, amides, such as N,N-dimethylformamide; sulfoxides, such as dimethylsulfoxide; ketones, such as acetone, methyl ethyl ketone, and methyl butyl ketone; aliphatic and aromatic hydrocarbons, such as hexane, octane, benzene, toluene, and the xylenes; esters, such as ethyl acetate; water; and the like. When the molecular includant is a cyclodextrin, particularly suitable solvents are the amides and sulfoxides.

For some applications, the colored composition of the present invention typically will be utilized in particulate form. In other applications, the particles of the composition should be very small. For example, the particles of a colored composition adapted for use as a toner in an electrophotographic process typically consist of 7-15 micrometer average diameter particles, although smaller or larger particles can be employed. It is important to note that the particles should be as uniform in size as possible. Methods of forming such particles are well known to those having ordinary skill in the art.

Photochemical processes involve the absorption of light quanta, or photons, by a molecule, e.g., the ultraviolet radiation transorber, to produce a highly reactive electronically excited state. However, the photon energy, which is proportional to the wavelength of the radiation, cannot be absorbed by the molecule unless it matches the energy difference between the unexcited, or original, state and an excited state. Consequently, while the wavelength range of the ultraviolet radiation to which the colored composition is exposed is not directly of concern, at least a portion of the radiation must have wavelengths which will provide the necessary energy to raise the ultraviolet radiation transorber to an energy level which is capable of interacting with the colorant.

It follows, then, that the absorption maximum of the ultraviolet radiation transorber ideally will be matched with the wavelength range of the ultraviolet radiation in order to increase the efficiency of the mutation of the colorant. Such efficiency also will be increased if the wavelength range of the ultraviolet radiation is relatively narrow, with the maximum of the ultraviolet radiation transorber coming within such range. For these reasons, especially suitable ultraviolet radiation has a wavelength of from about 100 to about 375 nanometers. Ultraviolet radiation within this range desirably may be incoherent, pulsed ultraviolet radiation from a dielectric barrier discharge excimer lamp.

The term "incoherent, pulsed ultraviolet radiation" has reference to the radiation produced by a dielectric barrier discharge excimer lamp (referred to hereinafter as "excimer lamp"). Such a lamp is described, for example, by U. Kogelschatz, "Silent discharges for the generation of ultraviolet and vacuum ultraviolet excimer radiation," Pure & Appl. Chem., 62, No. 9, pp. 1667-1674 (1990); and E. Eliasson and U. Kogelschatz, "UV Excimer Radiation from Dielectric-Barrier Discharges," Appl. Phys. B, 46, pp. 299-303 (1988). Excimer lamps were developed originally by ABB Infocom Ltd., Lenzburg, Switzerland. The excimer lamp technology since has been acquired by Heraeus Noblelight AG, Hanau, Germany.

The excimer lamp emits radiation having a very narrow bandwidth, i.e., radiation in which the half width is of the order of 5-15 nanometers. This emitted radiation is incoherent and pulsed, the frequency of the pulses being dependent upon the frequency of the alternating current power supply which typically is in the range of from about 20 to about 300 kHz. An excimer lamp typically is identified or referred to by the wavelength at which the maximum intensity of the radiation occurs, which convention is followed throughout this specification. Thus, in comparison with most other commercially useful sources of ultraviolet radiation which typically emit over the entire ultraviolet spectrum and even into the visible region, excimer lamp radiation is substantially monochromatic.

Excimers are unstable molecular complexes which occur only under extreme conditions, such as those temporarily existing in special types of gas discharge. Typical examples are the molecular bonds between two rare gaseous atoms or between a rare gas atom and a halogen atom. Excimer complexes dissociate within less than a microsecond and, while they are dissociating, release their binding energy in the form of ultraviolet radiation. Known excimers, in general, emit in the range of from about 125 to about 360 nanometers, depending upon the excimer gas mixture.

The amount or dosage level of ultraviolet radiation that the colorant of the present invention is exposed to will generally be that amount which is necessary to mutate the colorant. The amount of ultraviolet radiation necessary to mutate the colorant can be determined by one of ordinary skill in the art using routine experimentation. Power density is the measure of the amount of radiated electromagnetic power traversing a unit area and is usually expressed in watts per centimeter squared (W/cm²). The power density level range is between approximately 5 mW/cm² and 15 mW/cm², more particularly 8 to 10 mW/cm². The dosage level, in turn, typically is a function of the time of exposure and the intensity or flux of the radiation source which irradiates the colored composition. The latter is effected by the distance of the composition from the source and, depending upon the wavelength range of the ultraviolet radiation, can be effected by the atmosphere between the radiation source and the composition. Accordingly, in some instances it may be appropriate to expose the composition to the radiation in a controlled atmosphere or in a vacuum, although in general neither approach is desired.

The colored composition of the present invention may be utilized on or in any substrate. If the composition is present in a substrate, however, the substrate should be substantially transparent to the ultraviolet radiation which is employed to mutate the colorant. That is, the ultraviolet radiation will not significantly interact with or be absorbed by the substrate. As a practical matter, the composition typically will be placed on a substrate, with the most common substrate being paper. Other substrates, including, but not limited to, woven and nonwoven webs or fabrics, films, and the like, can be used, however.

By way of example, the composition of the present invention may be incorporated into a toner adapted to be utilized in an electrophotographic process. The toner includes the colorant, functionalized molecular includant, and a carrier. The carrier may be a polymer, and the toner may further contain a charge carrier. Briefly described, the electrophotographic process involves the steps of creating an image on a photoreceptor surface, applying toner to the photoreceptor surface to form a toner image which replicates the image, transferring the toner image to a substrate, and fixing the toner image to the substrate. After the toner has been fixed on the substrate, the colorant in the composition is mutated by irradiating the substrate with ultraviolet radiation at a dosage level sufficient to irreversibly mutate the colorant. For example, the ultraviolet radiation used in the method to mutate the colorant may have wavelengths of from about 100 to about 375 nanometers. As another example, the ultraviolet radiation may be incoherent, pulsed ultraviolet radiation produced by a dielectric barrier discharge excimer lamp.

When the colored composition is adapted to be utilized as a toner in an electrophotographic process, the composition also will contain a carrier, the nature of which is well known to those having ordinary skill in the art. For many applications, the carrier will be a polymer, typically a thermosetting or thermoplastic polymer, with the latter being the more common.

Examples of thermoplastic polymers suitable for the production of a toner include, but are not limited to, endcapped polyacetals, such as poly(oxymethylene) or polyformaldehyde, poly(trichloroacetaldehyde), poly(n-valeraldehyde), poly(acetaldehyde), poly(propionaldehyde), and the like; acrylic polymers, such as polyacrylamide, poly(acrylic acid), poly(methacrylic acid), poly(ethyl acrylate), poly(methyl methacrylate), and the like; fluorocarbon polymers, such as poly(tetrafluoroethylene), perfluorinated ethylenepropylene copolymers, ethylenetetrafluoroethylene copolymers, poly(chlorotrifluoroethylene), ethylene-chlorotrifluoroethylene copolymers, poly(vinylidene fluoride), poly(vinyl fluoride), and the like; epoxy resins, such as the condensation products of epichlorohydrin and bisphenol A; polyamides, such as poly(6-aminocaproic acid) or poly(ε-caprolactam), poly(hexamethylene adipamide), poly(hexamethylene sebacamide), poly(11-aminoundecanoic acid), and the like; polyaramides, such as poly(imino-1,3-phenyleneiminoisophthaloyl) or poly(m-phenylene isophthalamide), and the like; parylenes, such as poly-p-xylylene, poly(chloro-p-xylene), and the like; polyaryl ethers, such as poly(oxy-2,6-dimethyl-1,4-phenylene) or poly (p-phenylene oxide), and the like; polyaryl sulfones, such as poly(oxy-1,4-phenylenesulfonyl-1,4-phenyleneoxy-1,4-phenyleneisopropylidene-1,4-phenylene), poly(sulfonyl-1,4-phenyleneoxy-1,4-phenylenesulfonyl-4,4-biphenylene), and the like; polycarbonates, such as poly(bisphenol A) or poly(carbonyldioxy-1,4-phenyleneisopropylidene-1,4-phenylene), and the like; polyesters, such as poly(ethylene terephthalate), poly(tetramethylene terephthalate), poly(cyclohexylene-1,4-dimethylene terephthalate)or poly(oxymethylene-1,4-cyclohexylenemethyleneoxyterephthaloyl), and the like; polyaryl sulfides, such as poly(p-phenylene sulfide) or poly(thio-1,4-phenylene), and the like; polyimides, such as poly(pyromellitimido-1,4-phenylene), and the like; polyolefins, such as polyethylene, polypropylene, poly(1-butene), poly(2-butene), poly(1-pentene), poly(2-pentene), poly (3-methyl-1-pentene), poly(4-methyl-1-pentene), 1,2-poly-1,3-butadiene, 1,4-poly-1,3-butadiene, polyisoprene, polychloroprene, polyacrylonitrile, poly(vinyl acetate), poly(vinylidene chloride), polystyrene, and the like; and copolymers of the foregoing, such as acrylonitrile-butadienestyrene (ABS) copolymers, styrene-n-butylmethacrylate copolymers, ethylene-vinyl acetate copolymers, and the like.

Some of the more commonly used thermoplastic polymers include styrene-n-butyl methacrylate copolymers, polystyrene, styrene-n-butyl acrylate copolymers, styrene-butadiene copolymers, polycarbonates, poly(methyl methacrylate), poly(vinylidene fluoride), polyamides (nylon-12), polyethylene, polypropylene, ethylene-vinyl acetate copolymers, and epoxy resins.

Examples of thermosetting polymers include, but are not limited to, alkyd resins, such as phthalic anhydride-glycerol resins, maleic acid-glycerol resins, adipic acid-glycerol resins, and phthalic anhydride-pentaerythritol resins; allylic resins, in which such monomers as diallyl phthalate, diallyl isophthalate diallyl maleate, and diallyl chlorendate serve as nonvolatile cross-linking agents in polyester compounds; amino resins, such as aniline-formaldehyde resins, ethylene urea-formaldehyde resins, dicyandiamide-formaldehyde resins, melamine-formaldehyde resins, sulfonamide-formaldehyde resins, and urea-formaldehyde resins; epoxy resins, such as crosslinked epichlorohydrin-bisphenol A resins; phenolic resins, such as phenol-formaldehyde resins, including Novolacs and resols; and thermosetting polyesters, silicones, and urethanes.

In addition to the colorant and functionalized molecular includant, and optional carrier, the colored composition of the present invention also may contain additional components, depending upon the application for which it is intended. For example, a composition which is to be utilized as a toner in an electrophotographic process optionally may contain charge carriers, stabilizers against thermal oxidation, viscoelastic properties modifiers, cross-linking agents, plasticizers, and the like. Further, a composition which is to be utilized as a toner in an electrophotographic process optionally may contain charge control additives such as a quaternary ammonium salt; flow control additives such as hydrophobic silica, zinc stearate, calcium stearate, lithium stearate, polyvinylstearate, and polyethylene powders; and fillers such as calcium carbonate, clay and talc, among other additives used by those having ordinary skill in the art. For some applications, the charge carrier will be the major component of the toner. Charge carriers, of course, are well known to those having ordinary skill in the art and typically are polymer-coated metal particles. The identities and amounts of such additional components in the colored composition are well known to one of ordinary skill in the art.

When the colored composition is employed as a toner for an electrophotographic process, several variations are possible and come within the scope of the present invention. For example, a composition-based toner can be used to form a first image on a virgin paper sheet. The sheet then can be recycled by exposing the sheet to ultraviolet radiation in accordance with the present invention to render the colorant, and, as a consequence, the composition, colorless. A second image then can be formed on the sheet. The second image can be formed from a standard, known toner, or from a composition-based toner which is either the same as or different from the composition-based toner which was used to form the first image. If a composition-based toner is used to form the second image, the sheet can be recycled again, with the number of cycles being limited by the build-up of now colorless composition on the surface of the paper. Further, any subsequent image can be placed on either side of the sheet. That is, it is not required that a second image be formed on the side of the sheet on which the first image was formed.

In addition, the conversion of the composition-based toner image on the sheet to a colorless form does not have to take place on the sheet. For example, sheets having images formed from composition-based toners may be recycled in the traditional way. In place of the usual de-inking step, however, the sheets are exposed to ultraviolet radiation, either before or after being converted to pulp. Where the sheets are exposed to ultraviolet radiation after being converted to pulp, one of ordinary skill in the art would understand that no component of the pulp would interfere with the capability of the ultraviolet radiation transorber to mutate the colorant upon its exposure to ultraviolet radiation. The colorless toner then simply becomes incorporated into the paper formed from the resulting pulp.

The present invention also provides a method of making a functionalized molecular includant which includes the steps of providing a derivatized ultraviolet radiation transorber having a nucleophilic group, providing a derivatized molecular includant having more than two leaving groups per molecule, and reacting the derivatized ultraviolet radiation transorber with the derivatized molecular includant under conditions sufficient to result in the covalent coupling of an average of more than two ultraviolet radiation transorber molecules to each molecular includant molecule.

In general, the derivatized ultraviolet radiation transorber and the derivatized molecular includant are selected to cause the covalent coupling of the ultraviolet radiation transorber to the molecular includant by means of a bimolecular nucleophilic substitution reaction. Consequently, the choice of the nucleophilic group and the leaving groups and the preparation of the derivatized ultraviolet radiation transorber and derivatized molecular includant, respectively, may be readily accomplished by those having ordinary skill in the art without the need for undue experimentation.

The nucleophilic group of the derivatized ultraviolet radiation transorber may be any nucleophilic group capable of participating in a bimolecular nucleophilic substitution reaction, provided, of course, that the reaction results in the covalent coupling of more than two molecules of the ultraviolet radiation transorber to the molecular includant. The nucleophilic group generally will be a Lewis base, i.e., any group having an unshared pair of electrons. The group may be neutral or negatively charged. Examples of nucleophilic groups include, by way of illustration only, aliphatic hydroxy, aromatic hydroxy, alkoxides, carboxy, carboxylate, amino, and mercapto.

Similarly, the leaving group of the derivatized molecular includant may be any leaving group capable of participating in a bimolecular nucleophilic substitution reaction, again provided that the reaction results in the covalent coupling of more than two molecules of the ultraviolet radiation transorber to the molecular includant. Examples of leaving groups include, also by way of illustration only, p-toluenesulfonates (tosylates), p-bromobenzenesulfonates (brosylates) , p-nitrobenzenesulfonates (nosylates), methanesulfonates (mesylates), oxonium ions, alkyl perchlorates, ammonioalkane sulfonate esters (betylates), alkyl fluorosulfonates, trifluoromethanesulfonates (triflates), nonafluorobutanesulfonates (nonaflates), and 2,2,2-trifluoroethanesulfonates (tresylates).

The reaction of the derivatized ultraviolet radiation transorber with the derivatized molecular includant is carried out in solution. The choice of solvent depends upon the solubilities of the two derivatized species. As a practical matter, a particularly useful solvent is N,N-dimethylformamide (DMF).

The reaction conditions, such as temperature, reaction time, and the like generally are matters of choice based upon the natures of the nucleophilic and leaving groups. Elevated temperatures usually are not required. For example, the reaction temperature may be in a range of from about 0°C to around ambient temperature, i.e., to 20°-25°C.

The preparation of the functionalized molecular includant as described above generally is carried out in the absence of the colorant. However, the colorant may be associated with the derivatized molecular includant before reacting the derivatized ultraviolet radiation transorber with the derivatized molecular includant, particularly if a degree of substitution greater than about three is desired. When the degree of substitution is about three, it is believed that the association of the colorant with the functionalized molecular includant still may permit the colorant to be at least partially included in a cavity of the functionalized molecular includant. At higher degrees of substitution, such as about six, steric hindrance may partially or completely prevent the colorant from being at least partially included in a cavity of the functionalized molecular includant. Consequently, the colorant may be associated with the derivatized molecular includant which normally will exhibit little, if any, steric hindrance. In this instance, the colorant will be at least partially included in a cavity of the derivatized molecular includant. The above-described bimolecular nucleophilic substitution reaction then may be carried out to give a colored composition of the present invention in which the colorant is at least partially included in a cavity of the functionalized molecular includant.

The present invention is further described by the examples which follow. Such examples, however, are not to be construed as limiting in any way either the spirit or the scope of the present invention.

### Example 1

This example describes the preparation of the ultraviolet radiation transorber, 2-[p-(2-methyllactoyl)phenoxy]ethyl 1,3-dioxo-2-isoindolineacetate.

The following was admixed in a 250-ml, three-necked round bottomed flask fitted with a Dean & Stark adapter with condenser and two glass stoppers: 20.5 g (0.1 mole) of the wavelength selective sensitizer, phthaloylglycine (Aldrich Chemical Company, Inc., Milwaukee, Wisconsin); 24.6 g (0.1 mole) of the photoreactor, Irgacure® 2959 (Ciba-Geigy Corporation, Hawthorne, NY); 100 ml of benzene (Aldrich); and 0.4 g p-toluenesulfonic acid (Aldrich). The mixture was heated at reflux for 3 hours after which time 1.8 ml of water was collected. The solvent was removed under reduced pressure to give 40.1 g of white powder. The powder was recrystallized from 30% ethyl acetate in hexane (Fisher Scientific, Pittsburgh, Pennsylvania) to yield 38.1 g (93 percent) of a white crystalline powder having a melting point of 153-4°C. The resulting product, 2-[p- (2-methyllactoyl)phenoxy]ethyl 1,3-dioxo-2-isoindolineacetate, had the following physical parameters:
IR [Nujol Mull] υₘₐₓ 3440, 1760, 1740, 1680, 1600 cm⁻¹
¹HNMR [CDCl₃] ∂ppm 1.64[s], 4.25[m], 4.49[m], 6.92[m], 7.25[m], 7.86[m], 7.98[m], 8.06[m] ppm

### Example 2

This example describes the preparation of 2-[p-(2-methylallyloxy)phenoxy]ethyl 3-dioxo-2-isoindolineacetate.

To a 500-ml, round-bottomed flask fitted with a Dean & Stark adapter was added 20.0 g of the 2-[p-(2-methyllactoyl)phenoxy]ethyl 1,3-dioxo-2-isoindolineacetate obtained in Example 1, 0.1 g of p-toluenesulfonic acid (Aldrich), and 200 ml of benzene (Aldrich). The resulting mixture was heated at reflux temperature for 4 hours, during which time 0.8 ml of unter was collected. The solvent then was removed under reduced pressure in a rotary evaporator to yield 17.9 g of a white solid. The solid was purified on a silica gel column with 20 weight percent ethyl acetate in hexane as the eluant. Removal of the eluant left 17.6 g (92 percent) of 2-[p-(2-methylallyloxy)phenoxy]ethyl 1,3-dioxo-2-isoindolineacetate, m.p. 148-9°C. The material had the following physical parameter:
IR [Nujol Mull] ν_{**max**} 1760 cm⁻¹

### Example 3

This example describes the preparation of a derivatized ultraviolet radiation transorber, 2-[p-(2-methyl-2-mercaptomethylpropionyl)phenoxy]ethyl 1,3-dioxo-2-isoindolineacetate.

In a 500-ml, three-necked round-bottomed flask fitted with a bubbler tube, condenser, and addition funnel and containing a magnetic stirring bar was placed a solution of 10.0 g (0.025 mole) of the 2-[p-(2-methylallyloxy)phenoxy]ethyl 1,3-dioxo-2-isoindolineacetate obtained in Example 2 dissolved in 150 ml of anhydrous N,N-dimethylfornamide (DMF, Aldrich). The solution was cooled to 0°C in an ice bath while stirring with a magnetic stirrer. Hydrogen sulfide (Aldrich) then was bubbled into the solution for 30 minutes. The hydrogen sulfide-saturated solution was stirred at ambient temperature overnight while maintaining a positive hydrogen sulfide pressure in the flask. Excess hydrogen sulfide was removed from the solution and the flask by bubbling dry argon into the solution through the bubbler tube. The solution then was poured into ice water. The resulting mixture was extracted three times with 50-ml portions of diethyl ether (Aldrich). The ether extracts were combined, washed once with 100 ml of water, and dried over anhydrous magnesium sulfate (Aldrich). The ether was decanted from the magnesium sulfate and evaporated in a rotary evaporator. The solid which remained upon evaporation of the ether was purified as described in Example 2 to give 7.9 g (73 percent) of a white solid, 2-[p-(2-methyl-2-mercaptomethylpropionyl)phenoxy]ethyl 1,3-dioxo-2-isoindolineacetate, m.p. 160-1°C. The material had the following physical parameter:
IR [Nujol Mull] ν_{**max**} 2580 cm⁻¹

### Example 4

This example describes the preparation of a derivatized β-cyclodextrin.

To a 100-ml round-bottomed flask was added 6.0 g of β-cyclodextrin (American Maize Products Company, Hammond, Indiana), 10.0 g of p-toluenesulfonic acid (Aldrich), and 50 ml of a pH 10 buffer solution (Fisher). The contents of the flask were stirred at ambient temperature for eight hours, after which time it was poured over about 100 g of ice. The resulting mixture was extracted with ether. The aqueous phase was poured into 50 ml of acetone (Aldrich). The resulting cloudy mixture was filtered to give a white powder which was purified over a Sephadex® column (Aldrich) using a mixture of 5 parts of n-butanol, 4 parts of ethanol, and 3 parts of water (all by volume). Evaporation of the eluant left 10.9 g of a white powder.

The degree of substitution was determined by ¹³C NMR spectroscopy (DMF-d₆) by comparing the ratio of hydroxy-substituted carbons versus tosylated carbons, both at the 6 position. When the 6-position carbon bears a hydroxy group, the NMR peaks for each of the six carbon atoms are given in Table 1.

**Table 1**

| Carbon Atom | NMR Peak (ppm) |
|---|---|
| 1 | 101.8 |
| 2 | 72.9 |
| 3 | 72.3 |
| 4 | 81.4 |
| 5 | 71.9 |
| 6 | 59.8 |

The presence of the tosyl group shifts the NMR peaks of the 5-position and 6-position carbon atoms to 68.8 and 69.5 ppm, respectively.

The degree of substitution was calculated by integrating the NMR peak for the 6-position tosylated carbon, integrating the NMR peak for the 6-position hydroxy-substituted carbon, and dividing the former by the latter. The integrations yielded 23.6 and 4.1, respectively, and a degree of substitution of 5.9. Thus, the average degree of substitution in this example is about 6.

### Example 5

The procedure of Example 4 was repeated, except that the amounts of β-cyclodextrin and p-toluenesulfonic acid (Aldrich) were 6.0 g and 5.0 g, respectively. In this case, the degree of substitution was found to be about 3.

### Example 6

This example describes the preparation of a functionalized molecular includant from the derivatized ultraviolet radiation transorber of Example 3 and the derivatized molecular includant of Example 4.

To a 250-ml round-bottomed flask containing a magnetic stirring bar was added 10.0 g (4.8 mmoles) of the tosylated β-cyclodextrin prepared in Example 4, 20.7 g (48 mmoles) of the derivatized ultraviolet radiation transorber prepared in Example 3,2-[p-(2-methyl-2-mercaptomethylpropionyl)phenoxy]ethyl 1,3-dioxo-2-isoindolineacetate, and 100 ml of DMF. The resulting mixture was cooled to 0°C in an ice bath and stirred by means of a magnetic stirrer. To the mixture then was added dropwise a solution of 10 ml of N,N-diisopropylethylamine in 20 ml of DMF. The resulting reaction mixture was stirred at 0°C for eight hours. The mixture was poured over 100 g of ice and extracted with ether. To the aqueous phase was added 500 ml of acetone which caused a white precipitate to form. The precipitate was isolated by filtration and washed with acetone. The precipitate then was purified over a Sephadex column as described in Example 4. Evaporation of the eluant gave 16.7 g of functionalized β-cyclodextrin.

The degree of substitution was determined as described in Example 4. In this case, the presence of the derivatized ultraviolet radiation transorber shifts the NMR peak of the 6-position carbon atom to 63.1. The degree of substitution was calculated by integrating the NMR peak for the 6-position substituted carbon, integrating the NMR peak for the 6-position hydroxy-substituted carbon, and dividing the former by the latter. The integrations yielded 67.4 and 11.7, respectively, and a degree of substitution of 5.7. Thus, the average degree of substitution in this example is about 6.

### Example 7

The procedure of Example 6 was repeated, except that the derivatized molecular includant of Example 5 was employed in place of that from Example 4. The average degree of substitution was found to be about 3.

### Example 8

This example describes the preparation of a colored composition which includes a mutable colorant and the functionalized molecular includant from Example 6.

In a 250-ml Erlenmeyer flask containing a magnetic stirring bar was placed 20.0 g (5.4 mmoles) of the functionalized molecular includant obtained in Example 5 and 100 g of water. The water was heated to 80°C, at which temperature a clear solution was obtained. To the solution was added slowly, with stirring, 3.1 g (6.0 mmoles) of Victoria Pure Blue BO (Aldrich). A precipitate formed which was removed from the hot solution by filtration. The precipitate was washed with 50 ml of water and dried to give 19.1 g (84 percent) of a blue powder, a colored composition consisting of a mutable colorant, Victoria Pure Blue BO, and a molecular includant having covalently coupled to it an average of about six ultraviolet radiation transorber molecules per molecular includant molecule.

### Example 9

The procedure of Example 8 was repeated, except that the functionalized molecular includant from Example 7 was employed in place of that from Example 6.

### Example 10

This example describes the preparation of a β-cyclodextrin molecular includant having covalently bonded thereto about two ultraviolet radiation transorber molecules per molecule of molecular includant. The preparation utilized the 2-[p-(2-methylallyloxy)phenoxy]ethyl 1,3-dioxo-2-isoindolineacetate from Example 2.

The following were admixed in a 100-ml round-bottomed flask: 5.0 g (4 mmoles) of β-cyclodextrin (American Maize Products Company), 8.3 g (20 mmoles) of 2-[p-(2-methylallyloxy)phenoxy]ethyl 1,3-dioxo-2-isoindolineacetate from Example 2, 50 ml of anhydrous DMF, 20 ml of benzene, and 0.01 g of p-toluenesulfonyl chloride (Aldrich). The resulting mixture was chilled in an ice/salt bath and stirred for 24 hours. The mixture was poured into 150 ml of weak sodium bicarbonate solution and extracted three times with 50-ml portions of diethyl ether. The aqueous layer then was filtered to yield 9.4 g of a white solid. Reverse phase thin layer chromatography using a 50:50 by volume DMF:acetonitrile mixture showed a new product peak compared to the starting materials. By means of the procedure described in Examples 4 and 5, the product was shown to have an average degree of substitution of 2.

### Example 11

This example describes mutation or decolorization rates for the compositions of Examples 8, 9 and 10.

In each case, approximately 10 mg of the composition was placed on a steel plate (Q-Panel Company, Cleveland, Ohio). Three drops (about 0.3 ml) of acetonitrile (Burdick & Jackson, Muskegon, Michigan) was placed on top of the composition and the two materials were quickly mixed with a spatula and spread out on the plate as a thin film. Within 5-10 seconds of the addition of the acetonitrile, each plate was exposed to the radiation from a 222-nanometer excimer lamp assembly. The assembly consisted of a bank of four cylindrical lamps having a length of about 30 cm. The lamps were cooled by circulating water through a centrally located or inner tube of the lamp and, as a consequence, they operated at a relatively low temperature, i.e., about 50°C. The power density at the lamp's outer surface typically was in the range of from about 4 to about 20 joules per square meter (J/m²). However, such range in reality merely reflects the capabilities of current excimer lamp power supplies; in the future, higher power densities may be practical. The distance from the lamp to the sample being irradiated was 4.5 cm. The time for each film to become colorless to the eye was measured. The results are summarized in Table 2.

**Table 2**

| **Decolorization Times for Various Compositions** | |
|---|---|
| Composition | Decolorization Times (Seconds) |
| Example 8 | 1 |
| Example 9 | 3-4 |
| Example 10 | 7-8 |

While the data in Table 2 demonstrate the clear superiority of the colored compositions of the present invention, such data were plotted as degree of substitution versus decolorization time. The plot is shown in FIG. 1. FIG. 1 not only demonstrates the significant improvement of the colored compositions of the present invention when compared with compositions having a degree of substitution less than three, but also indicates that a degree of substitution of about 6 is about optimum. That is, the figure indicates that little if any improvement in decolorization time would be achieved with degrees of substitution greater than about 6.

### Example 12

This example describes the preparation of a complex consisting of a mutable colorant and the derivatized molecular includant of Example 4.

The procedure of Example 8 was repeated, except that the functionalized molecular includant of Example 6 was replaced with 10 g (4.8 mmoles) of the derivatized molecular includant of Example 4 and the amount of Victoria Pure Blue BO was reduced to 2.5 g (4.8 mmoles). The yield of washed solid was 10.8 g (86 percent) of a mutable colorant associated with a derivatized molecular includant, a tosylated β-cyclodextrin having an average of six tosyl groups per molecule of molecular includant.

### Example 13

This example describes the preparation of a colored composition which includes a mutable colorant and a functionalized molecular includant.

The procedure of Example 6 was repeated, except that the tosylated β-cyclodextrine was replaced with 10 g (3.8 mmoles) of the complex obtained in Example 12 and the amount of the derivatized ultraviolet radiation transorber prepared in Example 3 was 11.6 g (27 mmoles). The amount of colored composition obtained was 11.2 g (56 percent). The average degree of substitution was determined as described in Examples 4 and 6 and found to be 5.9, or about 6.

## Claims

1. A method of making a functionalized molecular includant which comprises the steps of:
providing a derivatized ultraviolet radiation transorber having a nucleophilic group;
providing a derivatized molecular includant having more than two leaving groups; and
reacting the derivatized ultraviolet radiation transorber with the derivatized molecular includant under conditions sufficient to result in the covalent coupling of an average of more than two ultraviolet radiation transorber molecules to each molecular includant molecule.

2. The method of claim 1, wherein the derivatized molecular includant has associated with it a mutable colorant.

3. The method of claim 1, wherein the nucleophilic group of the derivatized ultraviolet radiation transorber is a mercaptomethyl group.

4. The method of claim 1, wherein the leaving groups of the derivatized molecular includant are p-toluenesulfonyl groups.

5. The method of claim 4, wherein the derivatized molecular includant is a tosylated β-cyclodextrin.

6. The method of claim 1, wherein the nucleophilic group of the derivatized ultraviolet radiation transorber is a mercaptomethyl group and the leaving groups of the derivatized molecular includant are p-toluenesulfonyl groups.

7. The method of claim 1, wherein the derivatized ultraviolet radiation transorber is 2-[p-(2-methyl-2-mercaptomethylpropionyl)phenoxy]ethyl 1,3-dioxo-2-isoindolineacetate.

8. The method of claim 1, wherein the derivatized ultraviolet radiation transorber is 2-mercaptomethyl-2-methyl-4'-[2-[p-(3-oxobutyl)phenoxy]ethoxy]propiophenone.

9. The method of claim 8, wherein the derivatized molecular includant is a tosylated β-cyclodextrin having an average degree of substitution of from 3 to 21.

10. A derivatized molecular includant having more than two leaving groups capable of participating in a bimolecular nucleophilic substitution reaction, said includant having associated therewith a mutable colorant.

11. The derivatized molecular includant of claim 10, wherein the molecular includant has an average degree of substitution of from 3 to 21.

12. A tosylated β-cyclodextrin having associated therewith a mutable colorant.

13. The tosylated β-cyclodextrin of claim 12, wherein the average degree of substitution is greater than 2.

14. The tosylated β-cyclodextrin of claim 13, wherein the average degree of substitution is in a range of from 3 to 21.

15. A functionalized molecular includant available according to the method of any of claims 1 to 9.

16. A functionalized molecular includant comprising a molecular includant covalently bonded to from 3 to 21, with the exception of 5, ultraviolet radiation transorbers.

17. The functionalized molecular includant of claims 15 or 16, wherein the molecular includant is selected from the group consisting of clathrating agents, intercalate forming agents, zeolites and cyclodextrins.

18. The functionalized molecular includant of claim 17, wherein the cyclodextrin is a α-cyclodextrin, β-cyclodextrin, gamma-cyclodextrin, hydroxypropyl β-cyclodextrin, hydrohyethyl β-cyclodextrin, sulfated β-cyclodextrin or sulfated gamma-cyclodextrin.

19. A colored composition comprising:
a mutable colorant; and
the functionalized molecular includant of claims 15 or 16.

20. The colored composition of claim 19, wherein the colorant is at least partially included within a cavity of the molecular includant.

21. The colored composition of claim 19, wherein the ultraviolet radiation transorber absorbs ultraviolet radiation having a wavelength of from 100 to 375 nm.

## Patentansprüche

1. Verfahren zum Herstellen eines funktionalisierten Inhaltsstoffs, umfassend die Schritte:
Bereitstellen eines derivatisierten Ultraviolett-Strahlung-Transorbers mit einer nucleophilen Gruppe;
Bereitstellen eines derivatisierten Inhaltsstoffs mit mehr als zwei Austrittsgruppen; und
Reagieren des derivatisierten UV-Strahlung-Transorbers mit dem derivatisierten, molekularen Inhaltsstoff unter solchen Bedingungen, die ausreichen, um die kovalente Bindung von im Durchschnitt mehr als zwei UV-Strahlung-Transorbermolekülen mit jedem molekularen Inhaltsstoffmolekül zu ergeben.

2. Verfahren nach Anspruch 1, wobei sich der derivatisierte, molekulare Inhaltsstoff mit einem veränderbaren Farbstoff verbunden hat.

3. Verfahren nach Anspruch 1, wobei die nucleophile Gruppe des derivatisierten UV-Strahlung-Transorbers eine Mercaptomethylgruppe ist.

4. Verfahren nach Anspruch 1, wobei die Austrittsgruppen des derivatisierten, molekularen Inhaltsstoffs p-Toluolsulfonylgruppen sind.

5. Verfahren nach Anspruch 4, wobei der derivatisierte, molekulare Inhaltsstoff ein tosyliertes β-Cyclodextrin ist.

6. Verfahren nach Anspruch 1, wobei die nucleophile Gruppe des derivatisierten UV-Strahlung-Transorbers eine Mercaptomethylgruppe und die Austrittsgruppen des derivatisierten, molekularen Inhaltsstoffs p-Toluolsulfonylgruppen sind.

7. Verfahren nach Anspruch 1, wobei der derivatisierte UV-Strahlung-Transorber 2-[p-(2-Methyl-2-mercaptomethylpropionyl)phenoxy]ethyl-1,3-dioxo-2-isoindolinacetat ist.

8. Verfahren nach Anspruch 1, wobei der derivatisierte UV-Strahlung-Transorber 2-Mercaptomethyl-2-methyl-4'-[2-[p-(3-oxobutyl)phenoxy]ethoxy]propiophenon ist.

9. Verfahren nach Anspruch 8, wobei der derivatisierte, molekulare Inhaltsstoff ein tosyliertes β-Cyclodextrin mit einem Durchschnittssubstitutionswert von 3 bis 21 ist.

10. Derivatisierter, molekularer Inhaltsstoff mit mehr als zwei Austrittsgruppen, die in der Lage sind, an einer bimolekularen, nucleophilen Substitutionsreaktion teilzunehmen, wobei sich der Inhaltsstoff dabei mit einem veränderbaren Farbstoff verbunden hat.

11. Derivatisierter, molekularer Inhaltsstoff nach Anspruch 10, wobei der molekulare Inhaltsstoff einen Durchschnittssubstitutionswert von 3 bis 21 aufweist.

12. Tosyliertes β-Cyclodextrin, das sich dabei mit einem veränderbaren Farbstoff verbunden hat.

13. Tosyliertes β-Cyclodextrin nach Anspruch 12, wobei der Durchschnittssubstitutionswert größer als 2 ist.

14. Tosyliertes β-Cyclodextrin nach Anspruch 13, wobei der Durchschnittssubstitutionswert im Bereich von 3 bis 21 liegt.

15. Funktionalisierter, molekularer Inhaltsstoff, erhältlich gemäß dem Verfahren nach einem der Ansprüche 1 bis 9.

16. Funktionalisierter, molekularer Inhaltsstoff, umfassend einen molekularen Inhaltsstoff, kovalent von 3 bis 21, mit der Ausnahme von 5, an UV-Strahlung-Transorber gebunden.

17. Funktionalisierter, molekularer Inhaltsstoff nach Anspruch 15 oder 16, wobei der molekulare Inhaltstoff aus der Gruppe ausgewählt wurde, die aus Klathratierungsmitteln, interkalationsbildenden Mitteln, Zeolithen und Cyclodextrinen besteht.

18. Funktionalisierter, molekularer Inhaltsstoff aus Anspruch 17, wobei das Cyclodextrin ein α-Cyclodextrin, ein β-Cyclodextrin, ein gamma-Cyclodextrin, Hydroxypropyl-β-cyclodextrin, Hydroxyethyl-β-cyclodextrin, Sulfat-β-cyclodextrin oder Sulfat-gamma-cyclodextrin ist.

19. Gefärbte Zusammensetzung, umfassend:
einen veränderbaren Farbstoff; und
den funktionalisierten, molekularen Inhaltsstoff aus den Ansprüchen 15 oder 16.

20. Gefärbte Zusammensetzung aus Anpruch 19, wobei der Farbstoff zumindest teilweise innerhalb eines Hohlraums des molekularen Inhaltsstoffs enthalten ist.

21. Gefärbte Zusammensetzung aus Anspruch 19, wobei der UV-Strahlung-Transorber UV-Strahlung mit einer Wellenlänge von 100 bis 375 nm absorbiert.

## Revendications

1. Procédé de fabrication d'un composé fonctionnalisé d'inclusion moléculaire qui comprend les étapes suivantes :
l'apport d'un transorbeur de rayonnement ultraviolet dérivé ayant un groupe nucléophile ;
l'apport d'un composé dérivé d'inclusion moléculaire ayant plus de deux groupes labiles ; et
la réaction entre le transorbeur de rayonnement ultraviolet dérivé et le composé dérivé d'inclusion moléculaire dans des conditions suffisantes pour qu'il en résulte le couplage covalent d'en moyenne plus de deux molécules de transorbeur de rayonnement ultraviolet par molécule de composé à capacité d'inclusion moléculaire.

2. Procédé selon la revendication 1, dans lequel le composé dérivé d'inclusion moléculaire est associé à un colorant mutable.

3. Procédé selon la revendication 1, dans lequel le groupe nucléophile du transorbeur de rayonnement ultraviolet dérivé est un groupe mercaptométhyle.

4. Procédé selon la revendication 1, dans lequel les groupes labiles du composé dérivé d'inclusion moléculaire sont des groupes p-toluène-sulphonyle.

5. Procédé selon la revendication 4, dans lequel le composé dérivé d'inclusion moléculaire est une β-cyclodextrine tosylée.

6. Procédé selon la revendication 1, dans lequel le groupe nucléophile du transorbeur de rayonnement ultraviolet dérivé est un groupe mercaptométhyle et les groupes labiles du composé dérivé d'inclusion moléculaire sont des groupes p-toluène-sulphonyle.

7. Procédé selon la revendication 1, dans lequel le transorbeur de rayonnement ultraviolet dérivé est l'acétate de 2-[p-(2-méthyl-2-mercaptométhylpropionyl)phénoxy]éthyl 1,3-dioxo-2-isoindoline.

8. Procédé selon la revendication 1, dans lequel le transorbeur de rayonnement ultraviolet dérivé est la 2-mercaptométhyl-2-méthyl-4'-[2-[p-(3-oxobutyl)phénoxy] éthoxy]propiophénone.

9. Procédé selon la revendication 8, dans lequel le composé dérivé d'inclusion moléculaire est une β-cyclodextrine tosylée ayant un- degré moyen de substitution compris entre 3 et 21.

10. Composé dérivé d'inclusion moléculaire ayant plus de deux groupes labiles capables de participer à une réaction de substitution nucléophile bimoléculaire, ledit composé à capacité d'inclusion étant associé à un colorant mutable.

11. Composé dérivé d'inclusion moléculaire selon la revendication 10, dans lequel le composé à capacité d'inclusion moléculaire a un degré moyen de substitution compris entre 3 et 21.

12. β-cyclodextrine tosylée associée à un colorant mutable.

13. β-cyclodextrine tosylée selon la revendication 12, dans laquelle le degré moyen de substitution est supérieur à 2.

14. β-cyclodextrine tosylée selon la revendication 13, dans laquelle le degré moyen de substitution est compris dans la gamme allant de 3 à 21.

15. Composé fonctionnalisé d'inclusion moléculaire issu du procédé selon l'une quelconque des revendications 1 à 9.

16. Composé fonctionnalisé d'inclusion moléculaire comprenant un composé à capacité d'inclusion moléculaire lié de façon covalente à 3 à 21, à l'exception de 5, transorbeurs de rayonnement ultraviolet.

17. Composé fonctionnalisé d'inclusion moléculaire selon la revendication 15 ou 16, dans lequel le composé à capacité d'inclusion moléculaire est choisi dans le groupe consistant en les agents de clathratisation, les agents formant des produits d'insertions, les zéolites et les cyclodextrines.

18. Composé fonctionnalisé d'inclusion moléculaire selon la revendication 17, dans lequel la cyclodextrine est une α-cyclodextrine, β-cyclodextrine, gamma-cyclodextrine, hydroxypropyl-β-cyclodextrine, hydroxyéthyl-β-cyclodextrine, β-cyclodextrine sulfatée ou gamma-cyclodextrine sulfatée.

19. Composition colorée comprenant :
un colorant mutable ; et
le composé fonctionnalisé d'inclusion moléculaire selon la revendication 15 ou 16.

20. Composition colorée selon la revendication 19, dans laquelle le colorant est au moins partiellement inclus au sein d'une cavité du composé à capacité d'inclusion moléculaire.

21. Composition colorée selon la revendication 19, dans laquelle le transorbeur de rayonnement ultraviolet absorbe le rayonnement ultraviolet ayant une longueur d'onde comprise entre 100 et 375 nm.
